# EUROPEAN PATENT SPECIFICATION

(11) **EP 1 469 927 B1**
(45) Date of publication and mention of the grant of the patent: **18.06.2008**
(21) Application number: 02716053.0
(22) Date of filing: 24.01.2002
(51) Int. Cl.: B01D 11/02, A61K 35/66

(54) **METHOD AND DEVICE FOR PRODUCING PLANT EXTRACTS**
VERFAHREN UND VORRICHTUNG ZUR GEWINNUNG VON PFLANZENEXTRAKTEN
PROCEDE ET DISPOSITIF DE PRODUCTION D'EXTRAITS DE PLANTES

(43) Date of publication of application: 27.10.2004
(73) Proprietor: Primrose a.s., 110 00 Praha 1 (CZ)
(72) Inventor: VANA, Radek, 286 01 Caslav (CZ)
(74) Representative: Fischer, Michael
(86) International application number: PCT/CZ2002/000004
(87) International publication number: WO 2003/068360

(56) References cited:
- GB-A- 2 327 608
- US-A- 5 466 454

## Description

The invention relates to a method of production of plant extracts containing plant substances, especially for cosmetic products; according to the invention, the blend of plant materials is extracted with water and the extract is pasteurised and preserved. In addition, the invention pertains to equipment enabling to carry out this method.

So far the extracts of plant substances used to be prepared by maceration or extraction in ethanol or water and the respective extracted and macerated substances were only subsequently blended into the final extract and macerate. A disadvantage of this method consists in the fact that the extraction time, and especially maceration time, are long and besides that it is necessary to squeeze the material in order to release the respective substances in the case of maceration. Blending of the components is very time exacting and sophisticated equipment and large areas for the operation of such equipment are necessary. Another known method utilizes extractions using liquid nitrogen, which is very expensive, though. The disadvantages mentioned above have been partially removed by the production method of the cosmetic product extracts published in 1999, according to which the already blended individual plant substances are extracted together under the temperature 60 - 70°C during a 15 - 25 minute period. After the extraction pasteurisation, pH adjustment and preservation of the resulting extract should be carried out. A disadvantage of this method consists in the fact that the impurities and dry matters may only insufficiently be filtered off from the extracted plant substances. Therefore additional operations are necessary, namely withdrawal of the settled sediment and further filtration, for which another equipment is required. All the additional operations extend the production time. After pasteurisation it is necessary to cool the extract for those operations and, after they are carried out, the extract should be heated again in order to use the extract in further production operations. In this method of production of extracts containing plant substances either insufficient extraction of the required substances under lower temperatures or damage of the extracted substances under higher temperatures may occur. A necessary adjustment of the pH value, which occurs only subsequently, i.e. after pasteurisation, leads to an additional extension of the production time, which is then even extended by operation of the blending equipment. The aim of the invention is to remove the disadvantages of the existing technology status and to provide for a higher efficiency and lower power consumption connected with the method of production of extracts containing plant substances. Using this method, sufficiently pure and quality final product that does not require any subsequent adjustments, especially no filtration, no acidity adjustment and no repetitive heating could be obtained by extraction and pasteurisation only.

The disadvantages of the existing status of technology may be removed and the aim of the invention may be fulfilled by a method according to claim 1. In the starting stage of the phase extraction, the filtration bags containing the blend of the plant materials must be prepared for the respective operational phase extraction in accordance with the invention. For this purpose an initial phase is included into a procedure of the phase extraction; during this initial phase, a new blend of the plant material in a filtration bag is extracted in the first extraction vessel with water forming a partial extract, and a new blend of the plant material in the other filtration bag is extracted with the partial extract in the second extraction vessel forming a final extract. Under this advantageous method, the phase extraction includes at least one operation phase in which already extracted blend of the plant material in one filtration bag is extracted with water in an extraction vessel forming the partial extract, and new blend of the plant material in the other filtration bag is extracted in the extraction vessel, forming the final extract. Under another arrangement of the phase extraction, a filtration bag containing a new blend of the plant materials is extracted with water in an extraction vessel forming a partial extract; the filtration bag is then replaced with a second filtration bag containing a new blend of the plant materials, and the new blend of the plant materials placed in the second filtration bag is extracted by the partial extract forming the final extract. In this arrangement, the phase extraction includes at least a single additional phase in which the second filtration bag containing already extracted blend of the plant materials placed in the extraction vessel is extracted with water forming the partial extract, the second filtration bag is than replaced with the first filtration bag filled with a new blend of the plant materials and is extracted with the formed partial extract forming the final extract. In other advantageous arrangements the extraction may be performed using forced water and forced partial extract circulation, the temperature of the extracting medium at the entry to the extraction vessels may be maintained within the range 45 - 55 °C, preferably for the period 15 - 25 minutes, and the plant material blend may be extracted with water which pH acidity factor has been adjusted, the water acidity advantageously being within the pH range 6 - 8. Also, the output extract of the reservoir may be advantageously pasteurised within a period of 15 minutes and under the temperature ranging from 75 - 85 °C, under simultaneous filtration; during the pasteurisation the solution may be preserved by addition of preservatives within a 5-minute period and under the temperature 75 - 85 °C. Therefore, the pH-adjusted extracting water (lower pH in case of alkaloids, higher pH in case of saponins) passes through the first extraction vessel (in the first phase) under forced circulation. The input temperature of the extraction medium is 45 - 55 °C. In this first phase, the extraction is carried out under the above-mentioned temperature for the period of 15 - 25 minutes. In the second phase, the extraction is carried out in the second extraction vessel; the extracting medium is represented by the extract of the first phase. The extraction temperature is also 45 - 55 °C, the extraction time being also 15 - 25 minutes. After this second phase is over, the extract is transferred for pasteurisation. During the second phase of extraction with the partial extract, carried out in the first extraction vessel, the first phase of the water extraction in the second extraction vessel, i.e. the extraction of already extracted blend of the plant material placed in the same filtration bag and under the same temperature and time conditions, occurs. After the second phase of extraction with the partial extract in the first extraction vessel is over and after the first extraction vessel has been emptied, the first-phase partial extract of the second extraction vessel will be transferred to the first extraction vessel and a filtration bag containing a new blend of the plant material will be placed in the second extraction vessel. In the second extraction vessel the second extraction phase by.means of the partial extract and under the same temperature and time conditions is then carried out. After the second phase of extraction is over, the extraction bag containing already extracted plant material blend will be used for the first phase of water extraction. The filtration bag containing a new blend will be used for the subsequent second stage of extraction using the partial extract and the filtration bag containing the already extracted blend will be used for the fist stage of the water extraction. The order of utilization of the respective extraction vessels and filtration bags is mutually changed in such a way so that at first the extract and then water could pass through a filtration bag. This is naturally only relevant with an exception of the very first initiation of the extraction that may generally differ with the aim to prepare the initial condition of the plant material blend in the filtration bag for the respective method of the phase production of the extracts containing plant substances in accordance with the invention. The final extract is pasteurised under the temperature 75 - 85 °C for the period 20 minutes. The ratio between the plant material blend and the extracting water reaches 1:15 up to 1:25. The filtration consists of two phases. The first filtration is carried out during the extraction itself, by the passage through the filtration bag during the first and second extraction phases. The second filtration is carried out by means of a forced circulation closed circuit of the extract in the pasteurisation part, by passage through a filtration unit. The second filtration is a simultaneous process to the pasteurisation; both the processes start at the same time, however, the filtration period accounts for 15 minutes only. The product is preserved with the mixture of methylparabene, ethylparabene, propylparabene and sorbic acid dissolved in ethanol, by mixing into the ready extract during the period of pasteurisation after the filtration is over; in this second phase of filtration the filtration unit is excluded from the extract forced circulation and the forced circulation is switched over to the vessel. The preservatives are changed then, within the period of five minutes. The concentration of the preservatives is 0.25% (by weight) and that of ethanol 1% (by weight). The mixture of the preservatives and ethanol for the hair care product reaches up to 10% (by weight). The plant substances extract equipment according to the invention includes at least a single circuit in which an extraction vessel containing a filtration bag, a circulation pump and a heat exchanger are connected in series; in addition, the circuit includes a water reservoir with water supply and an input device capable of pH adjustment, and a final product reservoir, the water reservoir being connectable to the circuit, the circuit being connectable to the final product reservoir and the circuits being mutually connectable. Two circuits, in which the extraction vessel containing the filtration bag, circulating pump and heat exchanger are placed in series, may therefore be connected to the water reservoir equipped with water supply and a final product reservoir, the water reservoir being connectable to both the circuits and both the circuits being mutually connectable and connectable to the final product reservoir. The final product reservoir may be advantageously connected in series with a pump, a filtration unit and a heat exchanger, the circuit being connectable with the other circuits. Also, the preservative solution preparation vessel may be in parallel connected to the filtration unit. The extract production equipment therefore consists of a water reservoir equipped with an input device for pH adjustment that is connected to two circuits containing the extraction vessels with input filtration bags into which plant material blend may be placed. In addition, there are circulation pumps and a heat exchanger in the circuits. Further, the equipment consists of a circuit containing a final product reservoir, a circulation pump, a heat exchanger, a filtration unit and a preservative preparation vessel connected in parallel. The filtration unit consists of a filtration vessel containing a filtration bag, in which residual impurities and dry matter are deposited. The method of production of extracts containing plant substances according to the invention and the equipment for execution of such method provide for a time saving, power consumption decrease, efficiency increase, historical costs reduction, reduction of number of the necessary equipment and decrease of the demands on space. Using this method of production of extracts containing plant substances and the production equipment according to the invention, all required active substances may be extracted from the extractable blends of the plant material under a lower temperature of extraction, in required quality and without any risk of damage due to increased temperatures. The final extract with required pH value, which has already been adjusted during extraction, may thus be obtained. The final product shows a required purity degree, which is assured (especially by filtration) as early as during extraction and during pasteurisation. The method of production of extracts containing plant substances and the equipment according to the invention enable to obtain a ready preserved extract, which has already been preserved during pasteurisation. The two-phase extraction in two vessels, as mentioned above, may also be carried out using a single vessel by simple exchange of the filtration bags before the respective phases; the other vessel may be used for the purpose of pasteurisation.

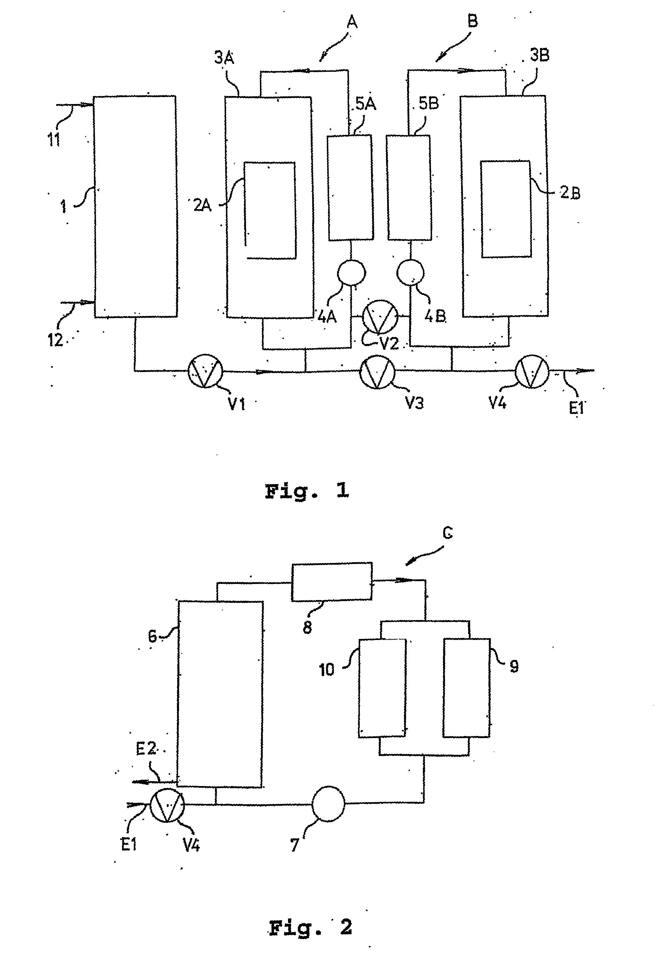

The invention will be further elucidated by means of a design, in which Fig. 1 represents the equipment for extraction of the plant substances and Fig. 2 part of the equipment serving for pasteurisation, filtration and preservation.

The course of the production method is as follows: in the starting stage, the plant material blend is placed in the filtration bags 2A and 2B and the bags are put in the first and second extraction vessels 3A and 3B. The extracting water, which pH has been adjusted to 7.5, circulates through the first filtration bag 2A placed in the first extracting vessel 3A. The ratio of the plant material and extracting water is 1:20; the temperature of the circulating extraction medium reaches 52 °C. The first phase of extraction takes 18 minutes. After the first phase is over, the partial extract is transferred into the second extraction vessel 3B, in which the partial extract represents the extracting medium circulating through the filtration bag 2B, into which a new plant material blend is placed. The new plant material blend and extracting water ratio is 1:20, the temperature of the circulating extraction medium being 52 °C. This second phase of extraction takes 18 minutes. After this second phase of extraction is over, the final extract is transferred into the final product reservoir 6. Then the phase extraction itself, according to the invention, begins. During the second phase of extraction in the second extraction vessel 3B, the first extraction phase is carried out in the extracting vessel 3A, using once leached plant material blend placed in the filtration bag 2A. In the second extraction vessel 3B the filling of the filtration bag 2B is replaced with a new one and the partial extract of the first extraction vessel is transferred into the second extraction vessel 3B. Then the second phase of extraction begins. After the second phase of extraction of the second extraction vessel 3B, which is carried out under the same thermal, ratio and time conditions, is over, the final extract is transferred into the final product reservoir 6. Meanwhile the first phase of extraction in the first extraction vessel 3A is carried out, the partial extract is transferred into the second extraction vessel 3B, the plant material blend of the filtration- bag 2A in the extraction vessel 3A is replaced with a new one and, subsequently, the second phase of water extraction takes place in the first extraction vessel 3A with the filtration bag kept from the previous extraction. In this way, the 3A and 3B vessels (from the semantic point of view, the first phase and the second phase) are swapped. The flow direction of the extracting water and extracting partial extract is always changed after the first and second phase of extraction are simultaneously carried out. The above-mentioned method of production of extracts containing plant substances may be modified for a single vessel only. According to such modification, the phase extraction may include an initial phase in which a filtration bag 2A containing a new mixture of plant material is extracted with water in the extraction vessel 3A forming the partial extract, then the filtration bag 2A is replaced with the second filtration bag 2B containing a new plant material blend, and the new plant material blend is extracted with the partial extract in the second filtration bag 2B forming the final extract. This arrangement of the phase extraction includes at least one additional operational phase in which the second filtration bag 2B containing the leached plant material blend placed in the extraction vessel 3A is extracted with water forming partial extract and the second filtration bag 2B is replaced with the first filtration bag 2A filled with a new plant material blend which is then leached with the already formed partial extract forming the final extract. The second part of the equipment (Fig. 2) is used for pasteurisation, filtration completion and preservation. The final extract, which has been transferred into the reservoir 6, circulates, under 80 °C temperature, through the filtration unit 9 and heat exchanger 8 by means of forced circulation induced by pump 7 for 15 minutes. The solution of preservatives in ethanol with concentrations 0.05% (by weight) propylparabene, 0.05% (by weight) ethylparabene, 0.05% (by weight) methylparabene and 0.1% (by weight) sorbic acid in 1% (by weight) ethanol are prepared in the preservative preparation vessel. After fifteen minutes of pasteurisation process, the filtration is interrupted by closure of the filtration circuit, for next 5 minutes the pasteurisation utilizes the preservative preparation vessel in order to mix the solution thoroughly. The final product is suitable for the production of complexion care cosmetic products. As for production of hair care cosmetic products, the production procedure is the same as in the previous case, however, p-H of the extracting water should be adjusted to 6.5. The plant material blend / extracting medium ratio reaches 1:15 and the ethanol content in the preservatives is 10% (by weight). The method of production of extracts utilizing the equipment according to the invention may be, without any additional modifications i.e. without filtration, preservation, pH adjustment or heating, used wherever concentrated pure plant extracts are required and wherever their addition is necessary (suitable for the cosmetic) medical products. The production equipment for the production of extracts containing plant substances includes, according to Fig.1, a water reservoir with a water supply input device 11 and a pH adjusting substances input device 12. The water reservoir 1 is in parallel connected to two circuits A, B for phase extraction of the plant substances blends. Both the circuits A and B contain the first / second extraction vessels 3A, 3B into which the filtration bags 2A, 2B are placed. The extraction tanks are placed in series with the circulation pumps 4A, 4B and heat exchangers 5A, 5B in both the A,B circuits. Both the A, B circuits are mutually connected at the level of the bottom of the extraction vessels 3A, 3B through a closing valve V2. In addition, the A, B circuits are interconnected through a closing valve V3, both the A, B circuits being connectable with the connecting line E1 for the final extract output, in which a closing valve is placed, through the V3 closing valve. According to Fig. 2, the connecting line E1 is connected to the final product reservoir 6 through a closing valve V4. The reservoir 6 is equipped with an output E2 for the outlet of the final product. The final product reservoir 6 is placed in the closed circuit C in series with a circulation pump 7, a heat exchanger 8 and a filtration unit 9, to which a vessel 10 for preparation of preservatives is connected in parallel. According to Fig. 1, the water reservoir 1 is connectable to the A, B circuits and the A and B circuits are mutually connectable and connectable to the final product reservoir 6. According to Fig. 2, in the C circuit the final product reservoir 6 may advantageously be connected in series with the circulation pump 7, filtration unit 9 and the heat exchanger 8, the C circuit being connectable with the circuits. Also, the preservative preparation vessel may advantageously be connected to the filtration unit. The filtration unit 9 consists of a filtration vessel with a filtration bag in which the residual impurities and dry matter deposit. In the equipment for extraction of plant substances according to Fig. 1, water acidity in the water reservoir 1 is first adjusted; the pH-adjusted water supplies the A circuit through the closing valve V1 and the B circuit through the closing valve V3. In both the circuits, extraction takes place as described above. The partial extract is mutually transferred between the respective A, B circuits through the V2 valve. The final extract may be drained from the A, B circuits using the connecting line E1, the V3 closing valve and the V4 closing valve into the circuit C (not drawn) containing the final product reservoir 6. As mentioned above, the phase extraction may be performed in a single circuit of the A, B circuits. In that case, for example, the filtration bag 2A containing leached plant material blend would be replaced with the filtration bag 2B containing a new plant material blend and the second circuit B would serve to another purpose. According to Fig. 2, the E1 connecting line supplies the C circuit, the final product reservoir 6, respectively, with the final extract. After closing the V4 closing valve, the final extract is set to circulation in the C circuit; the final extract then passes through the filtration unit 9 and heat exchanger 8 while a preservative is being added to the final extract from a preservative preparation vessel 10 connected in parallel. After the pasteurisation and the preservation is over, the final product is drained from the final product reservoir 2 by means of the E2 outlet.

## Claims

1. Method of extraction of plant substances to a final extract, wherein the final extract is pasteurised and preserved, and wherein
a plant substance blend is periodically extracted in extraction periods consisting of a first stage in which the plant substance blend in a first filtration bag (2A) is partially extracted with water to a partial extract and of a second stage in which the plant substance blend in a second filtration bag (2B) is extracted by the partial extract of the first stage to the final extract, wherein in the second filtration bag (2B) a new plant substance blend is extracted in all extraction periods and in the first filtration bag (2A) a new plant substance blend is extracted in the initial extraction period and a partially extracted plant substance blend of the second stage of the foregoing extraction period is extracted in the following operational extraction periods.

2. Method of extracting plant substances according to claim 1,
**characterised in that**
in the first stage of the initial extraction period the new plant substance blend of the first filtration bag (2A) is extracted by water to the partial extract in a first extraction vessel (3A), the partial extract is transferred to a second extraction vessel (3B) with the second filtration bag (2B) filled with a new plant substance blend, which is then extracted by the partial extract in the second extraction vessel (3B) to the final extract.

3. Method of extracting plant substances according to claim 1,
**characterised in that**
in the first stage of the operational extraction period the plant substance blend partially extracted in the second stage of the foregoing extraction period in the second filtration bag (2B) of a second extraction vessel (3B), is extracted in the second extraction vessel (3B) by water to the partial extract, the partial extract is transferred to a first extraction vessel (3A) with a first filtration bag (2A) filled with a new plant substance blend, which is extracted in the first extraction vessel (3A) by the partial extract to the final extract.

4. Method of production of plant extracts according to claim 1,
**characterised in that**
the extraction is carried out by means of a forced water and a forced partial extract circulation.

5. Method of production of plant extracts according to claim 1,
**characterised in that**
the temperature of the extracting medium at the input to the extraction vessels (3A, 3B) is maintained at 45 - 55 °C.

6. Method of production of plant extracts according to claim 1,
**characterised in that**
the extraction time of the extracting medium in the extraction vessels (3A, 3B) is maintained at 15 - 25 minutes.

7. Method of production of plant extracts according to claim 1,
**characterised in that**
the plant substance blend is extracted with adjusted acidity pH-factor water, whereas the water acidity is adjusted to pH = 6 - 8.

8. Method of production of plant extracts according to claims 1 - 7,
**characterised in that**
the final extract is pasteurised for the period of 15 minutes and under the temperature 75 - 85 °C under simultaneous filtration.

9. Method of production of plant extracts according to claim 8,
**characterised in that**
during pasteurisation, preservation with preservatives lasting 5 minutes takes place under the temperature 75 - 85 °C.

10. Device for production of plant extracts suitable for carrying out the method of claim 1, comprising
circuits (A, B) in which the extraction vessels (3A; 3B) containing filtration bags (2A; 2B) are placed in series with circulation pumps (4A; 4B) and heat exchangers (5A; 5B); by a water reservoir (1) with water supply (11) and with an input device (12) for pH value adjustment and by a final product reservoir (6), wherein the circuits (A, B) are connectable mutually and with the water reservoir (1) and with the final products reservoir (6).

11. Device for production of plant extracts according to claim 10,
**characterised in that**
the final product reservoir (6) is connected in series with a pump (7), a filtration unit (9) and a heat exchanger (8) in an accessory circuit (C), whereas the accessory circuit (C) is connectable to the circuits (A, B).

12. Device for production of plant extracts according to claim 11,
**characterised in that**
a reservoir (10) for preservative solution preparation is connected in parallel to the filtration unit (9).

## Patentansprüche

1. Das Verfahren zur Extraktion von Pflanzenstoffen zum Finalextrakt, das pasteurisiert und konserviert wird, wobei das Pflanzenstoffgemisch periodisch in Extraktionsperioden extrahiert wird, die aus einer ersten Phase, in der das Pflanzenstoffgemisch im ersten Filtrationsbeutel (2A) teilweise mit Wasser zu einem Teilauszug extrahiert wird, sowie einer zweiten Phase bestehen, in der das Pflanzenstoffgemisch im zweiten Filterbeutel (2B) mit dem Teilauszug der ersten Phase zum Finalextrakt extrahiert wird, wobei in dem zweiten Filterbeutel (2B) das neue Pflanzenstoffgemisch in allen Extraktionsperioden und in dem ersten Filterbeutel (2A) in der anfänglichen Extraktionsperiode das neue Pflanzenstoffgemisch und in den nachfolgenden Betriebsextraktionsperioden das partiell extrahierte Pflanzenstoffgemisch der zweiten Phase der vorherigen Extraktionsperiode extrahiert werden.

2. Das Verfahren der Herstellung von Pflanzenstoffextrakten nach Anspruch 1,
**gekennzeichnet dadurch, dass**
das neue Pflanzenstoffgemisch des ersten Filterbeutels (2A) in der ersten Phase der anfänglichen Extraktionsperiode im ersten Extrahiergefäß (3A) mit Wasser zu einem Teilauszug extrahiert wird, der Teilauszug in das zweite Extrahiergefäß (3B) mit dem zweiten Filterbeutel (2B) umgeleitet wird, der mit dem neuen Pflanzenstoffgemisch gefüllt ist, das danach im zweiten Extrahiergefäß (3B) mit dem Teilauszug zum Finalextrakt extrahiert wird.

3. Das Verfahren der Herstellung von Pflanzenstoffextrakten nach Anspruch 1,
**gekennzeichnet dadurch, dass**
in der ersten Phase der Betriebsextraktionsperiode, das teilweise in der zweiten Phase der vorherigen Extraktionsperiode im zweiten Filterbeutel (2B) des zweiten Extrahiergefäßes (3B) extrahierte Pflanzenstoffgemisch im zweiten Extrahiergefäß (3B) mit Wasser zu einem Teilextrakt extrahiert wird und der Teilauszug in das erste Extrahiergefäß (3A) mit dem ersten Filterbeutel (2A) geleitet wird, der mit dem neuen Pflanzenstoffgemisch befüllt ist, das in dem ersten Extrahiergefäß (3A) mit dem Teilauszug zum Finalextrakt extrahiert wird.

4. Das Verfahren der Herstellung von Pflanzenstoffextrakten nach Anspruch 1,
**gekennzeichnet dadurch, dass**
das Extrahieren im Zwangsumlauf des Wassers und Teilauszugs erfolgt.

5. Das Verfahren der Herstellung von Pflanzenstoffextrakten nach Anspruch 1,
**gekennzeichnet dadurch, dass**
die Temperatur des Extraktionsmediums am Zugang zu den Extrahiergefäßen (3A, 3B) auf dem Wert von 45 - 55°C gehalten wird.

6. Das Verfahren der Herstellung von Pflanzenstoffextrakten nach Anspruch 1,
**gekennzeichnet dadurch, dass**
die Verweildauer des Mediums in den Extrahiergefäßen (3A, 3B) auf dem Wert von 15 - 25 Minuten gehalten wird.

7. Das Verfahren der Herstellung von Pflanzenstoffextrakten nach Anspruch 1,
**gekennzeichnet dadurch, dass**
das Pflanzenstoffgemisch mit Wasser mit eingestelltem pH Säurefaktor extrahiert wird, wobei der Säuregrad des Wasser auf pH = 6 - 8 eingestellt wird.

8. Das Verfahren der Herstellung von Pflanzenstoffextrakten nach Ansprüchen 1 - 7,
**gekennzeichnet dadurch, dass**
das Finalextrakt für die Dauer von 15 Minuten bei einer Temperatur von 75 - 85°C bei gleichzeitiger Filtration pasteurisiert wird.

9. Das Verfahren der Herstellung von Pflanzenstoffextrakten nach Anspruch 8,
**gekennzeichnet dadurch, dass**
im Verlauf der Pasteurisierung die Konservierung mit Konservierungsstoffen für die Dauer von 5 Minuten und bei einer Temperatur von 75 - 85°C erfolgt.

10. Anlage zur Herstellung von Pflanzenstoffauszügen, die zur Ausführung der Herstellungsweise nach Anspruch 1 aufgebaut ist und umfassend Kreisläufe (A, B), in denen die Extrahiergefäße (3A, 3B) mit Filtersäcken (2A; 2B) hintereinander und in Reihe mit Umlaufpumpen (4A; 4B) und Wärmetauschern (5A, 5B) geschaltet sind; Wasservorratsbehälter (1) mit Wasserzufuhr (11) und Zugang (12) zur Aufbereitung des pH-Wertes und Vorratsbehälter (6) des Finalprodukts, wobei die Kreisläufe (A, B) untereinander und mit dem Wasservorratsbehälter (1) und Vorratsbehälter (6) des Finalprodukts vereinbar sind.

11. Anlage zur Herstellung von Pflanzenstoffauszügen nach Anspruch 10,
**gekennzeichnet dadurch, daß**
der Vorratsbehälter (6) des Finalprodukts mit der Pumpe (7), der Filtereinheit (9) und dem Wärmeaustauscher (8) im Nebenkreislauf (C) in Reihe geschaltet ist, wobei der Nebenkreislauf (C) mit den Kreisläufen (A, B) verbunden werden kann.

12. Anlage zur Herstellung von Pflanzenstoffauszügen nach Anspruch 10,
**gekennzeichnet dadurch, daß**
der Vorratsbehälter (10) zur Aufbereitung der Lösung der Konservierungsstoffe parallel zur Filtereinheit (9) geschaltet ist.

## Revendications

1. Méthode d'extraction de substances végétales pour les transformer en un extrait final qui est pasteurisé et conservé en sachant que le mélange de substances végétales est lessivé périodiquement dans des périodes d'extraction composées d'une première phase où le mélange de substances végétales est partiellement lessivé à l'eau dans un premier sac filtrant (2A) et est ainsi transformé en extrait partiel, et d'une seconde phase durant laquelle le mélange de substances végétales est lessivé avec l'extrait partiel de la première phase dans un second sac filtrant (2B) et est ainsi transformé en extrait final, en sachant que dans le second sac filtrant (2B), le nouveau mélange de substances végétales est lessivé durant toute les périodes d'extraction et que dans le premier sac filtrant (2A), le nouveau mélange de substances végétales est lessivé durant la période d'extraction de départ et lors des périodes d'extraction suivantes, on lessivera le mélange de substances végétales partiellement lessivé provenant de la seconde phase de la période d'extraction précédente.

2. Méthode de production d'extraits de substances végétales selon la revendication 1,
**caracterisée par le fait que**
durant la première phase de la période d'extraction de départ, le nouveau mélange de substances végétales du premier sac filtrant (2A) est lessivé à l'eau pour être transformé en extrait partiel dans un premier récipient d'extraction (3A), l'extrait partiel est ensuite amené dans un second récipient d'extraction (3B) équipé d'un second sac filtrant (2B), rempli par le nouveau mélange de substances végétales qui est ensuite lessivé par l'extrait partiel dans un second récipient d'extraction (3B) pour être transformé en extrait final.

3. Méthode de production d'extraits de substances végétales selon la revendication 1,
**caracterisée par le fait que**
durant la première phase de la période d'extraction, le mélange de substances végétales, partiellement lessivé durant la seconde phase de la période d'extraction précédente dans le second bac filtrant (2B) du second récipient d'extraction (3B), est lessivé à l'eau dans le second récipient d'extraction (3B) et est ainsi transformé en extrait partiel. Cet extrait partiel est ensuite amené dans le premier récipient d'extraction (3A) dont le premier sac filtrant (2A) est rempli par le nouveau mélange de substances végétales qui est lessivé par l'extrait partiel dans le premier récipient d'extraction (3A) pour être transformé en extrait final.

4. Méthode de production d'extraits de substances végétales selon la revendication 1,
**caracterisée par le fait que**
l'extraction est réalisée avec circulation forcée pour l'eau et pour l'extrait partiel.

5. Méthode de production d'extraits de substances végétales selon la revendication 1,
**caracterisée par le fait que**
la température du fluide de lessivage à l'entrée des récipients d'extraction (3A, 3B) est maintenue à une valeur de 45 - 55 °C.

6. Méthode de production d'extraits de substances végétales selon la revendication 1,
**caracterisée par le fait que**
la durée de séjour du fluide de lessivage dans les récipients d'extraction (3A, 3B) est maintenue à une valeur de 15 - 25 minutes.

7. Méthode de production d'extraits de substances végétales selon la revendication 1,
**caracterisée par le fait que**
le mélange de substances végétales est lessivé avec une eau dont le facteur d'acidité pH a été modifié, en sachant que l'acidité de l'eau est adaptée en vue d'obtenir une valeur de pH = 6 - 8.

8. Méthode de production d'extraits de substances végétales selon les revendication 1-8,
**caracterisée par le fait que**
l'extrait final est pasteurisé durant 15 minutes à une température de 75 - 85 °C simultanément à la filtration.

9. Méthode de production d'extraits de substances végétales selon la revendication 9,
**caracterisée par le fait que**
dans le courant de la pasteurisation, on réalise une conservation à l'aide de produits de conservation et ce, durant 5 minutes à une température de 75 - 85 °C.

10. Dispositif de production d'extraits de substances végétales adapté pour obtenir une méthode selon la revendication 1, comprenant des circuits (A, B) dans lesquels se trouvent des récipients d'extraction (3A; 3B) contenant des sacs filtrants (2A; 2B) raccordés en série avec des pompes de circulation (4A; 4B) et des échangeurs de chaleur (5A, 5B); un réservoir d'eau (1) avec alimentation en eau (11) et une entrée (12) pour la modification de la valeur du pH et un réservoir (6) contenant le produit final, en sachant que les circuits (A, B) peuvent être raccordés l'un avec l'autre, ainsi qu'avec le réservoir d'eau (1) et avec le réservoir de produit final (6).

11. Disposifif de production d'extraits de substances végétales selon la revendicatin 10,
**caracterisée par le fait que**
le réservoir du produit final (6) est raccordé en série avec une pompe (7), une unité filtrante (9) et un échangeur de chaleur (8) dans le circuit adjacent (C), en sachant que le circuit adjacent (C) peut être raccordé aux circuits (A, B).

12. Dispositif de production d'extraits de substances végétales selon la revendication 11,
**caracterisée par le fait que**
le réservoir (10) pour la préparation de la solution de produits de conservation est raccordé en parallèle avec l'unité filtrante (9).
